# EUROPÄISCHE PATENTSCHRIFT

(11) **EP 1 110 453 B1**
(45) Veröffentlichungstag und Bekanntmachung des Hinweises auf die Patenterteilung: **02.05.2003**
(21) Anmeldenummer: 01107639.5
(22) Anmeldetag: 13.05.1996
(51) Int. Cl.: A01N 43/56, A01N 43/653, C07D 249/12, C07D 401/04

(54) **Triazolyloxybenzyl-alkoxyacrylsäureester, Verfahren zu ihrer Herstellung und ihre Verwendung**
Triazolyloxybenzyl alkoxyacrylic acid esters, process for producing them and their use
Esters d'acide triazolyloxybenzyl-alcoxyacrylique, leur procédé de fabrication et leur utilisation

(30) Priorität: 24.05.1995 DE 19519041
(43) Veröffentlichungstag der Anmeldung: 27.06.2001
(62) Teilanmeldung aus: 96916055.5
(73) Patentinhaber: BASF AKTIENGESELLSCHAFT, 67056 Ludwigshafen (DE)
(72) Erfinder: Müller, Bernd, Dr., 67227 Frankenthal (DE); Kirstgen, Reinhard, Dr., 67434 Neustadt (DE); König, Hartmann, Dr., 69115 Heidelberg (DE); Rack, Michael, Dr., 69123 Heidelberg (DE); Oberdorf, Klaus, Dr., 69117 Heidelberg (DE); Röhl, Franz, Dr., 67105 Schifferstadt (DE); Sauter, Hubert, Dr., 68167 Mannheim (DE); Lorenz, Gisela, Dr., 67434 Hambach (DE); Ammermann, Eberhard, Dr., 64646 Heppenheim (DE); Strathmann, Siegfried, Dr., 67117 Limburgerhof (DE); Harries, Volker, Dr., 67227 Frankenthal (DE)
(74) Vertreter: Fitzner, Uwe, Dr.

(56) Entgegenhaltungen:
- EP-A- 0 299 694
- WO-A-94/00436

## Beschreibung

Die vorliegende Erfindung betrifft Verbindungen der allgemeinen Formel I 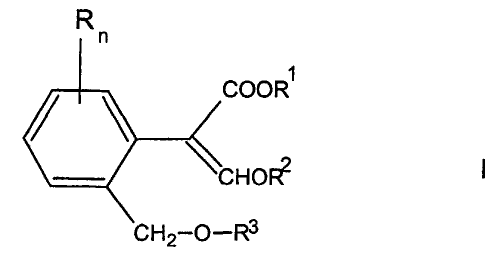 in der der Index und die Substituenten die folgende Bedeutung haben:
- n: 0, 1, 2, 3 oder 4, wobei die Substituenten R verschieden sein können, wenn n größer als 1 ist;
- R: Nitro, Cyano, Halogen,
C₁-C₁₀-Alkyl, C₂-C₁₀-Alkenyl, C₂-C₁₀-Alkinyl, C₁-C₁₀-Alkoxy, C₂-C₁₀-Alkenyloxy, C₂-C₁₀-Alkinyloxy
wobei die aliphatischen Gruppen partiell oder vollständig halogeniert sein können und/oder einen bis drei, insbesondere einen, der folgenden Reste tragen können:
Nitro, Cyano, C₁-C₄-Alkoxy, C₁-C₄-Alkoxycarbonyl oder eine Gruppe =N-OR^{x}, in der R^{x} für C₁-C₆-Alkyl, C₃-C₆-Alkenyl und C₃-C₆-Alkinyl steht,
oder ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann,
welches wiederum partiell oder vollständig halogeniert sein kann und/oder einen bis drei, der folgenden Reste tragen kann: Nitro, Cyano, C₁-C₄-Alkyl, C₁-C₄-Alkoxy und C₁-C₄-Alkoxycarbonyl;
- R¹,R²: C₁-C-Alkyl;
- R³: ein Triazolrest der Formel A 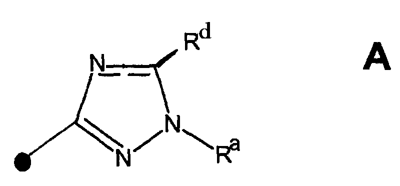
wobei die mit • gekennzeichnete Bindung der Bindung zum Sauerstoff entspricht und in denen die Substituenten die folgende Bedeutung haben:
- R^{a}: C₁-C₁₀-Alkyl, C₂-C₁₀-Alkenyl oder C₂-C₁₀-Alkinyl;
wobei die aliphatischen Gruppen und/oder einen bis drei, insbesondere einen, der folgenden Reste tragen können:
Nitro, Cyano, C₁-C₄-Alkoxy, C₁-C₄-Alkoxycarbonyl oder eine Gruppe =N-OR^{x}, in der R^{x} für C₁-C₆-Alkyl, C₃-C₆-Alkenyl und C₃-C₆-Alkinyl steht,
oder ein ein- oder zweikerniges aromatisches Ringsystem, welches neben Kohlenstoffatomen ein bis vier Stickstoffatome oder ein oder zwei Stickstoffatome und ein oder zwei Sauerstoff- oder Schwefelatome oder ein Sauerstoff- oder Schwefelatom als Ringglieder enthalten kann,
welches wiederum partiell oder vollständig halogeniert sein kann und/oder einen bis drei der folgenden Reste tragen kann: Nitro, Cyano, C₁-C₄-Alkyl, C₁-C₄-Alkoxy und C₁-C₄-Alkoxycarbonyl;
- R^{d}: Wasserstoff, Cyano, Halogen, C₁-C₁₀-Alkyl, C₁-C₁₀-Alkoxy, C₂-C₁₀-Alkenyl, C₂-C₁₀-Alkenyloxy, C₂-C₁₀-Alkinyl, C₂-C₁₀-Alkinyloxy ein gesättigter oder ein- oder zweifach ungesättigter Ring, welcher neben Kohlenstoffatomen ein bis drei der folgenden Heteroatome als Ringglieder enthalten kann, der partiell oder vollständig halogeniert sein kann und/oder einen bis drei der folgenden Reste tragen kann:
Nitro, Cyano, C1-C4-Alkyl, C1-C4-Alkoxy und C1-C4-Alkoxycarbonyl Sauerstoff, Schwefel und Stickstoff, und welcher direkt oder über ein Sauerstoff oder Schwefelatom an das Gerüst gebunden sein kann;
oder ein partiell oder vollständig halogenierter und/ oder durch einen bis drei der folgenden Reste: Nitro, Cyano, C₁-C₄-Alkyl, C₁-C₄-Alkoxy und C₁-C₄-Alkoxycarbonyl substituierter ein- oder zweikerniger aromatischer Rest, welcher neben Kohlenstoffatomen ein bis vier Stickstoffatome oder ein oder zwei Stickstoffatome und ein oder zwei Sauerstoff- oder Schwefelatome oder ein Sauerstoff- oder ein Schwefelatom als Ringglieder enthalten kann.

Ausserdem betrifft die Erfindung Verfahren zur Herstellung dieser Verbindungen sowie sie enthaltende Mittel und deren Verwendung zur Bekämpfunf von tierischen Schädlingen und Schadpilzen.

In der Literatur werden Hetaryloxybenzyl-alkoxyacrylsäureester mit fungizider und z.T. auch insektizider, acarizider und nematodizider Wirkung in allgemeiner Form beschrieben (EP-A 278 595; EP-A 254 426; EP-A 358 692; EP-A- 299 694; WO-A 94/19,331, WO-A 94/00,436).

Der vorliegenden Erfindung lagen demgegenüber Verbindungen mit verbesserter Wirksamkeit als Aufgabe zugrunde.

Demgemäß wurden die eingangs definierten Verbindungen I gefunden. Außerdem wurden Verfahren zur Herstellung dieser Verbindungen sowie sie enthaltende Mittel und deren Verwendung zur Bekämpfung von tierischen Schädlingen und Schadpilzen gefunden.

Die Verbindungen I sind auf verschiedenen aus der genannten Literatur an sich bekannten Methoden erhältlich.

Beispielsweise erhält man die Verbindungen I durch Umsetzung des Benzylderivats II mit einem Hydroxyazol der Formel III in Gegenwart einer Base.

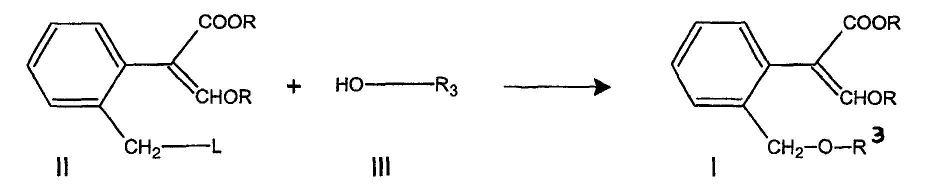

L in der Formel II bedeutet eine nucleophil austauschbare Gruppe, beispielsweise Halogen, z.B. Chlor, Brom und Iod, oder ein Alkyl- oder Arylsulfonat, z.B. Methylsulfonat, Trifluormethylsulfonat, Phenylsulfonat und 4-Methylphenylsulfonat.

Die Umsetzung wird üblicherweise bei Temperaturen von 0°C bis 100°C, vorzugsweise 20°C bis 60°C, durchgeführt.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, i-Propanol, n-Butanol und tert.-Butanol, Ketone wie Aceton und Methylethylketon sowie Essigsäureethylester, Dimethylsulfoxid, Dimethylformamid, N-Methylpyrrolidon, Dimethylacetamid, 1,3-Dimethylimidazolidin-2-on und 1,2-Dimethyltetrahydro-2(1H)-pyrimidin, vorzugsweise Methylenchlorid, Aceton, N-Methylpyrrolidon und Dimethylformamid. Es können auch Gemische der genannten Lösungsmittel verwendet werden.

Als Basen kommen allgemein anorganische Verbindungen wie Alkalimetall- und Erdalkalimetallhydroxide (z.B. Lithiumhydroxid, Natriumhydroxid, Kaliumhydroxid und Calziumhydroxid), Alkalimetall- und Erdalkalimetalloxide (z.B. Lithiumoxid, Natriumoxid, Calziumoxid und Magnesiumoxid), Alkalimetall- und Erdalkalimetallhydride (z.B. Lithiumhydrid, Natriumhydrid, Kaliumhydrid und Calziumhydrid), Alkalimetallamide (z.B. Lithiumamid, Natriumamid und Kaliumamid), Alkalimetall- und Erdalkalimetallcarbonate (z.B. Lithiumcarbonat, Kaliumcarbonat und Calziumcarbonat) sowie Alkalimetallhydrogencarbonate (z.B. Natriumhydrogencarbonat), metallorganische Verbindungen, insbesondere Alkalimetallalkyle (z.B. wie Methyllithium, Butyllithium und Phenyllithium), Alkylmagnesiumhalogenide (z.B. Methylmagnesiumchlorid) sowie Alkali-metall- und Erdalkalimetallalkoholate (z.B. Natriummethanolat, Natriumethanolat, Kaliumethanolat, Kalium-tert.-Butanolat und Dimethoxymagnesium), außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Natriummethanolat Kaliumcarbonat, Kaliummethanolat und Kaliumtert.-butanolat.

Die Basen werden im allgemeinen äquimolar, im Überschuß oder gegebenenfalls als Lösungsmittel verwendet.

Es kann für die Umsetzung vorteilhaft sein, eine katalytische Menge eines Kronenethers (z.B. 18-Krone-6 oder 15-Krone-5) zuzusetzen.
Die Umsetzung kann auch in Zweiphasensystemen bestehend aus einer Lösung von Alkali- oder Erdalkalihydroxiden oder -carbonaten in Wasser und einer organischen Phase (z.B. aromatische und/oder halogenierte Kohlenwasserstoffe) durchgeführt werden. Als Phasentransferkatalysatoren kommen hierbei beispielsweise Ammoniumhalogenide und -tetrafluoroborate (z.B. Benzyltriethylammoniumchlorid, Benzyltributylammoniumbromid, Tetrabutylammoniumchlorid, Hexadecyltrimethylammoniumbromid oder Terabutylammoniumtetrafluoroborat) sowie Phosphoniumhalogenide (z.B. Tetrabutylphosphoniumchlorid und Tetraphenylphosphoniumbromid) in Betracht.

Es kann für die Umsetzung vorteilhaft sein, zunächst das Hydroxyazol III mit der Base in das entsprechende Hydroxylat umzusetzen, welchen dann mit dem Benzylderivat umgesetzt wird.

Die für die Herstellung der Verbindungen I benötigten Ausgangsstoffe II sind aus der eingangs zitierten Literatur bekannt oder können nach den dort beschriebenen Verfahren hergestellt werden. Die Ausgangsstoffe III sind aus der Literatur bekannt oder können nach den dort beschriebenen Verfahren hergestellt werden 3-Hydroxytriazole: Chem. Ber. 56, 1794 (1923); DE-A 21 50 169; DE-A 22 00 436; US-A 4,433,148; J. Med. Chem. 33, 2772 (1990); Synthesis 1987, 986; DE-A 22 60 015; DE-A 24 17 970].

Des weiteren erhält man die Verbindungen I durch Umsetzung von α-Ketoestern der Formel IVc im Sinne einer Wittig- oder Wittig-Horner Reaktion {beispielsweise mit (C₆H₅)₃P⁺-CH₂OR² Cl⁻} gemäß der folgenden Reaktionsgleichung (vgl. EP-A 534 216). 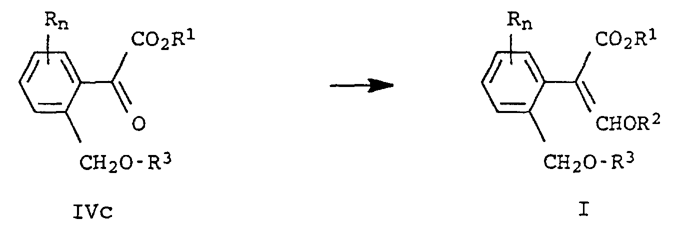 Die Ketoester können analog bekannten Verfahren erhalten werden [vgl. EP-A 493 711; Synth. Commun. 21, 2045 (1991); Synth. Commun. 11, 943 (1981)].

Außerdem können die Verbindungen I dadurch erhalten werden, daß man ein Nitril der Formel IVa mit einem Alkohol (R¹OH) zunächst gemäß EP-A 493 711 in den entsprechenden Benzylester IVb überführt und IVb anschließend gemäß EP-A 203 608 in I überführt.

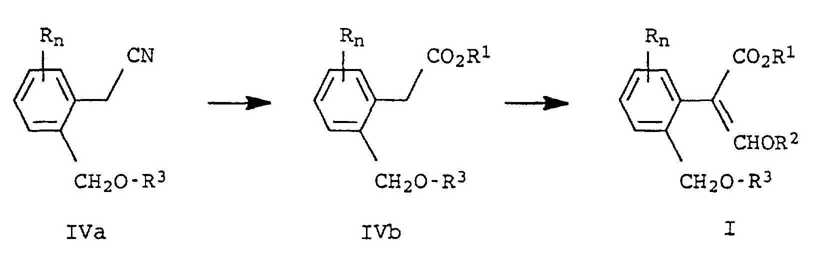

Die Herstellung der Nitrile IVa ist in EP-A 596 692 beschrieben.

Die Verbindungen I können in Bezug auf die Doppelbindung sowohl in der E- als auch in der Z-Konfiguration vorliegen. Beide Isomere können erfindungsgemäß gemeinsam oder getrennt angewendet werden. Bevorzugt ist insbesondere das E-Isomer (Konfiguration bezüglich der Carboxylat-Alkoxy-Gruppe).

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit 1 bis 4 Kohlenstoffatomen, z.B. Methyl, Ethyl, Propyl, 1-Methylethyl, Butyl, 1-Methyl-propyl, 2-Methylpropyl und 1,1-Dimethylethyl;
Halogenalkyl: geradkettige oder verzweigte Alkylgruppen mit 1 bis 4 Kohlenstoffatomen (wie vorstehend genannt), wobei diese in Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
ggf. subst. Alkyl: gesättigte, geradkettige oder verzweigte Kohlenwasserstoffreste, insbesondere mit 1 bis 10 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-l-methylpropyl und 1-Ethyl-2-methylpropyl;
ggf. subst. Alkenyl: ungesättigte, geradkettige oder verzweigte Kohlenwasserstoffreste, insbesondere mit 2 bis 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-l-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-l-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-l-methyl-2-propenyl, 1-Ethyl-2-methyl-1-propenyl und 1-Ethyl-2-methyl-2-propenyl;
ggf. subst. Alkinyl: geradkettige oder verzweigte Kohlenwasserstoffgruppen, insbesondere mit 2 bis 20 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl- 1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
ein ggf. subst. gesättigter oder ein- oder zweifach ungesättigter Ring, welcher neben Kohlenstoffatomen ein bis drei der folgenden Heteroatome als Ringglieder enthalten kann: Sauerstoff, Schwefel und Stickstoff, beispielsweise Carbocyclen wie Cyclopropyl, Cyclopentyl, Cyclohexyl, Cyclopent-2-enyl, Cyclohex-2-enyl, 5- bis 6-gliedrige, gesättigte oder ungesättigte Heterocyclen, enthaltend ein bis drei Stickstoffatome und/oder ein Sauerstoff- oder Schwefelatom wie 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-l-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl, vorzugsweise 2-Tetrahydrofuranyl, 2-Tetrahydrothienyl, 2-Pyrrolidinyl, 3-Isoxazolidinyl, 3-Isothiazolidinyl, 1,3,4-Oxazolidin-2-yl, 2,3-Dihydrothien-2-yl, 4,5-Isoxazolin-3-yl, 3-Piperidinyl, 1,3-Dioxan-5-yl, 4-Piperidinyl, 2-Tetrahydropyranyl, 4-Tetrahydropyranyl;
oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann, d.h. Arylreste wie Phenyl und Naphthyl, vorzugsweise Phenyl oder 1- oder 2-Naphthyl, und Hetarylreste, beispielsweise 5-Ring Heteroaromaten enthaltend ein bis drei Stickstoffatome und/oder ein Sauerstoff- oder Schwefelatom wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,5-Triazol-3-yl, 1,2,3-Triazol-4-yl, 1,2,3-Triazol-5-yl, 1,2,3-Triazol-4-yl, 5-Tetrazolyl, 1,2,3,4-Thiatriazol-5-yl und 1,2,3,4-Oxatriazol-5-yl, insbesondere 3-Isoxazolyl, 5-Isoxazolyl, 4-Oxazolyl, 4-Thiazolyl, 1,3,4-Oxadiazol-2-yl und 1,3,4-Thiadiazol-2-yl;
Sechsring-Heteroaromaten enthaltend ein bis vier Stickstoffatome als Heteroatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl, insbesondere 2-Pyridinyl, 3-Pyridinyl, 4-Pyridinyl, 2-Pyrimidinyl, 4-Pyrimidinyl, 2-Pyrazinyl und 4-Pyridazinyl.

Der Zusatz "ggf. subst" in Bezug auf Alkyl-, Alkenyl- und Alkinylgruppen soll zum Ausdruck bringen, daß diese Gruppen partiell oder vollständig halogeniert sein können (d.h. die Wasserstoffatome dieser Gruppen können teilweise oder vollständig durch gleiche oder verschiedene Halogenatome wie vorstehend genannt (vorzugsweise Fluor, Chlor und Brom, insbesondere Fluor und Chlor) ersetzt sein können und/oder einen bis drei, insbesondere einen, der folgenden Reste tragen können:
Nitro, Cyano, C₁-C₄-Alkoxy, C₁-C₄-Alkoxycarbonyl oder eine Gruppe =N-OR^{x}, in der R^{x} für C₁-C₆-Alkyl, C₃-C₆-Alkenyl und C₃-C₆-Alkinyl steht (R^{x} ist vorzugsweise C₁-C₄-Alkyl),
oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann, d.h. Arylreste wie Phenyl und Naphthyl, vorzugsweise Phenyl oder 1- oder 2-Naphthyl, und Hetarylreste, beispielsweise 5-Ring Heteroaromaten enthaltend ein bis drei Stickstoffatome und/oder ein Sauerstoff- oder Schwefelatom wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,5-Triazol-3-yl, 1,2,3-Triazol-4-yl, 1,2,3-Triazol-5-yl, 1,2,3-Triazol-4-yl, 5-Tetrazolyl, 1,2,3,4-Thiatriazol-5-yl und 1,2,3,4-Oxatriazol-5-yl, insbesondere 3-Isoxazolyl, 5-Isoxazolyl, 4-Oxazolyl, 4-Thiazolyl, 1,3,4-Oxadiazol-2-yl und 1,3,4-Thiadiazol-2-yl;
Sechsring-Heteroaromaten enthaltend ein bis vier Stickstoffatome als Heteroatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl, insbesondere 2-Pyridinyl, 3-Pyridinyl, 4-Pyridinyl, 2-Pyrimidinyl, 4-Pyrimidinyl, 2-Pyrazinyl und 4-Pyridazinyl.

Der Zusatz *"ggf*. *subst"* in Bezug auf die cyclischen (gesättigten, ungesättigtern oder aromatischen) Gruppen soll zum Ausdruck bringen, daß diese Gruppen partiell oder vollständig halogeniert sein können (d.h. die Wasserstoffatome dieser Gruppen können teilweise oder vollständig durch gleiche oder verschiedene Halogenatome wie vorstehend genannt (vorzugsweise Fluor, Chlor und Brom, insbesondere Fluor und Chlor) ersetzt sein können und/oder einen bis drei, der folgenden Reste tragen können: Nitro, Cyano, C₁-C₄-Alkyl, C₁-C₄-Alkoxy und C₁-C₄-Alkoxycarbonyl.

Die bei den Resten genannten ein- oder zweikernigen aromatischen oder heteroaromatischen Systeme können ihrerseits partiell oder vollständig halogeniert sein, d.h. die Wasserstoffatome dieser Gruppen können partiell oder vollständig durch Halogenatome wie Fluor, Chlor, Brom und Jod, vorzugsweise Fluor und Chlor ersetzt sein.

Diese ein- oder zweikernigen aromatischen oder heteroaromatischen Systeme können neben den bezeichneten Halogenatomen zusätzlich ein bis drei der folgenden Substituenten tragen:
Nitro, Cyano, Thiocyanato;
Alkyl, besonders C₁-C₆-Alkyl wie vorstehend genannt, vorzugsweise Methyl, Ethyl, 1-Methylethyl, 1,1-Dimethylethyl, Butyl, Hexyl, insbesondere Methyl und 1-Methylethyl;
C₁-C₄-Halogenalkyl, wie vorstehend genannt, vorzugsweise Trichlormethyl, Difluormethyl, Trifluormethyl, 2,2-Difluorethyl, 2,2,2-Trifluorethyl und Pentafluorethyl;
C₁-C₄-Alkoxy, vorzugsweise Methoxy, Ethoxy, 1-Methylethoxy und 1,1-Dimethylethoxy, insbesondere Methoxy;
C₁-C₄-Halogenalkoxy;
C₁-C₄-Alkylthio;
C₁-C₄-Alkylamino;
Di-C₁-C₄-alkylamino, insbesondere N,N-Dimethylamino;
C₁-C₆-Alkylcarbonyl, insbesondere Ethylcarbonyl;
C₁-C₆-Alkoxycarbonyl, insbesondere Ethoxycarbonyl;
C₁-C₆-Alkylaminocarbonyl, insbesondere Methylaminocarbonyl;
Di-C₁-C₆-alkylaminocarbonyl, insbesondere N,N-Dimethylaminocarbonyl;
C₁-C₆-Alkylcarboxyl, insbesondere Methylcarboxyl und 1,1-Dimethylethylcarboxyl;
C₁-C₆-Alkylcarbonylamino, insbesondere Ethylcarbonylamino;
C₁-C₆-Alkylcarbonyl-C₁-C₆-alkylamino, insbesondere Methylcarbonyl-methylamino;
C₃-C₇-Cycloalkyl wie Cyclopropyl, Cyclobutyl, Cyclopentyl, Cyclohexyl und Cycloheptyl, vorzugsweise Cyclopropyl, Cyclopentyl und Cyclohexyl, insbesondere Cyclopropyl;
weitere Reste für ggf. subst. ein- oder zweikernige aromatische oder heteroaromatische Reste:
Alkenyl, Alkinyl, Halogenalkenyl, Halogenalkinyl, Alkenyloxy, Alkinyloxy, Halogenalkenyloxy, Halogenalkinyloxy, Alkenylthio, Alkinylthio, Alkylsulfoxy, Alkylsulfonyl, Alkenylsulfoxy, Alkinylsulfoxy, Alkinylsulfonyl,
eine Gruppe C(R^{y})=N-OR^{x}, in der R^{x} und R^{y} für C₁-C₆-Alkyl, C₃-C₆-Alkenyl und C₃-C₆-Alkinyl stehen, R^{x} und R^{y} sind bevorzugt C₁-C₄-Alkyl, Cycloalkenyl, Cycloalkenyloxy, Cycloalkenylthio, Cycloalkenylamino.

Im Hinblick auf ihre biologische Wirksamkeit sind Verbindungen I bevorzugt, in denen R¹ und R² C₁-C₂-Alkyl, insbesondere Methyl, bedeuten.

Des weiteren werden Verbindungen I bevorzugt, in denen n für 0 oder 1, insbesondere 0, steht.

Für den Fall, daß n für 1 steht, werden Verbindungen I bevorzugt, in denen R für eine der folgenden Gruppen steht: Fluor, Chlor, Cyano, Methyl, Trifluormethyl oder Methoxy.

Außerdem werden Verbindungen I, in denen n für 1 steht, bevorzugt, in denen R in der 3- oder 6-Position zum Acrylatrest steht.

Daneben werden Verbindungen I bevorzugt, in denen R^{a} für ggf. subst. C₁-C₄-Alkyl oder C₃-C₆-Cycloalkyl steht.

Gleichermaßen werden Verbindungen I bevorzugt, in denen R^{a} für einen ggf. subst. ein- oder zweikernigen aromatischen oder heteroaromatischen Rest steht.

Des weiteren werden Verbindungen I bevorzugt, in denen R^{a} für einen ggf. subst. 6-Ring-Heteroaromaten, insbesondere Pyridin und Pyrimidin, steht.

Ebenfalls bevorzugt sind Verbindungen I, in denen R^{a} für einen ggf. subst. aromatischen Rest, insbesondere Phenyl, steht.

Insbesondere sind solche Verbindungen I bevorzugt, in denen R^{a} für ggf. substituiertes Phenyl oder Benzyl steht. Als Substituenten des Phenylrestes kommen in diesen Fällen bevorzugt Halogen, Cyano, C₁-C₄-Alkyl, C₁-C₄-Alkoxy, C₁-C₂-Halogenalkyl, C₁-C₂-Halogenalkoxy, C₁-C₄-Alkoxycarbonyl, Phenyl und Oxy-C₁-C₂-alkylidenoxy in Betracht.

Ebenfalls bevorzugt sind Verbindungen I, in denen R^{a} für einen ggf. substituierten Sechsring-Heteroaromaten wie Pyridyl und Pyrimidyl steht. Als Substituenten des Sechsring-Heteroaromaten kommen bevorzugt Cyano, Halogen, C₁-C₄-Alkyl, C₁-C₂-Halogenalkyl, C₁-C₄-Alkoxy, C₁-C₂-Halogenalkoxy und Phenyl in Betracht.

Außerdem werden Verbindungen I bevorzugt, in denen R^{d} für Wasserstoff steht.

Gleichermaßen werden Verbindungen I bevorzugt, in denen R^{d} für Halogen, C₁-C₄-Alkyl, C₁-C₄-Halogenalkyl, C₁-C₄-Alkoxy und C₁-C₄-Halogenalkoxy, insbesondere Chlor, Methyl, Trifluormethyl und Methoxy, steht.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet (unabhängig von der Kombination, in der sie genannt sind) eine besonders bevorzugte Ausgestaltung des betreffenden Substituenten dar.

### Tabelle 1

Verbindungen der allgemeinen Formel I.1, in denen Rₙ für Wasserstoff steht, R^{d} für Wasserstoff steht und die Kombination des Index x und der Gruppe R^{x} für eine Verbindung jeweils einer Zeile der Tabelle A entspricht

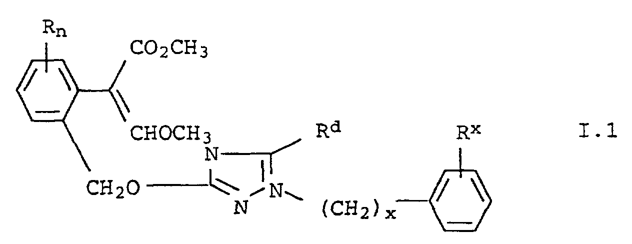

### Tabelle 2

Verbindungen der allgemeinen Formel I.1, in denen Rₙ für Wasserstoff steht, R^{d} für Methyl steht und die Kombination des Index x und der Gruppe R^{x} für eine Verbindung jeweils einer Zeile der Tabelle A entspricht

### Tabelle 3

Verbindungen der allgemeinen Formel I.1, in denen Rₙ für Wasserstoff steht, R^{d} für Chlor steht und die Kombination des Index x und der Gruppe R^{x} für eine Verbindung jeweils einer Zeile der Tabelle A entspricht

### Tabelle 4

Verbindungen der allgemeinen Formel I.1, in denen Rₙ für Wasserstoff steht, R^{d} für Methoxy steht und die Kombination des Index x und der Gruppe R^{x} für eine Verbindung jeweils einer Zeile der Tabelle A entspricht

### Tabelle 5

Verbindungen der allgemeinen Formel I.1, in denen Rₙ für 3-Chlor steht, R^{d} für Wasserstoff steht und die Kombination des Index x und der Gruppe R^{x} für eine Verbindung jeweils einer Zeile der Tabelle A entspricht

### Tabelle 6

Verbindungen der allgemeinen Formel I.1, in denen Rₙ für 3-Chlor steht, R^{d} für Methyl steht und die Kombination des Index x und der Gruppe R^{x} für eine Verbindung jeweils einer Zeile der Tabelle A entspricht

### Tabelle 7

Verbindungen der allgemeinen Formel I.1, in denen Rₙ für 3-Chlor steht, R^{d} für Chlor steht und die Kombination des Index x und der Gruppe R^{x} für eine Verbindung jeweils einer Zeile der Tabelle A entspricht

### Tabelle 8

Verbindungen der allgemeinen Formel I.1, in denen Rₙ für 3-Chlor steht, R^{d} für Methoxy steht und die Kombination des Index x und der Gruppe R^{x} für eine Verbindung jeweils einer Zeile der Tabelle A entspricht

**Tabelle A**

| **Nr.** | **R**^{**x**} | **X** |
|---|---|---|
| 1 | H | O |
| 2 | 2-F | O |
| 3 | 3-F | O |
| 4 | 4-F | O |
| 5 | 2,4-F₂ | O |
| 6 | 2,4,6-F₃ | O |
| 7 | 2,3,4,5,6-F₅ | O |
| 8 | 2,3-F₂ | O |
| 9 | 2-Cl | O |
| 10 | 3-Cl | O |
| 11 | 4-Cl | O |
| 12 | 2,3-Cl₂ | O |
| 13 | 2,4-Cl₂ | O |
| 14 | 2,5-Cl₂ | O |
| 15 | 2,6-Cl₂ | O |
| 16 | 3,4-Cl₂ | O |
| 17 | 3,5-Cl₂ | O |
| 18 | 2,3,4-Cl₃ | O |
| 19 | 2,3,5-Cl₃ | O |
| 20 | 2,3,6-Cl₃ | O |
| 21 | 2,4,5-Cl₃ | O |
| 22 | 2,4,6-Cl₃ | O |
| 23 | 3,4,5-Cl₃ | O |
| 24 | 2,3,4,6-Cl₄ | O |
| 25 | 2,3,5,6-Cl₄ | O |
| 26 | 2,3,4,5,6-Cl₅ | O |
| 27 | 2-Br | O |
| 28 | 3-Br | O |
| 29 | 4-Br | O |
| 30 | 2,4-Br₂ | O |
| 31 | 2,5-Br₂ | O |
| 32 | 2,6-Br₂ | O |
| 33 | 2,4,6-Br₃ | O |
| 34 | 2,3,4,5,6-Br₅ | O |
| 35 | 2-J | O |
| 36 | 3-J | O |
| 37 | 4-J | O |
| 38 | 2,4-J₂ | O |
| 39 | 2-Cl, 3-F | O |
| 40 | 2-Cl, 4-F | O |
| 41 | 2-Cl, 5-F | O |
| 42 | 2-Cl, 6-F | O |
| 43 | 2-Cl, 3-Br | O |
| 44 | 2-Cl, 4-Br | O |
| 45 | 2-Cl, 5-Br | O |
| 46 | 2-Cl, 6-Br | O |
| 47 | 2-Br, 3-Cl | O |
| 48 | 2-Br, 4-Cl | O |
| 49 | 2-Br, 5-Cl | O |
| 50 | 2-Br, 3-F | O |
| 51 | 2-Br, 4-F | O |
| 52 | 2-Br, 5-F | O |
| 53 | 2-Br, 6-F | O |
| 54 | 2-F, 3-Cl | O |
| 55 | 2-F, 4-Cl | O |
| 56 | 2-F, 5-Cl | O |
| 57 | 3-Cl, 4-F | O |
| 58 | 3-Cl, 5-F | O |
| 59 | 3-Cl, 4-Br | O |
| 60 | 3-Cl, 5-Br | O |
| 61 | 3-F, 4-Cl | O |
| 62 | 3-F, 4-Br | O |
| 63 | 3-Br, 4-Cl | O |
| 64 | 3-Br, 4-F | O |
| 65 | 2,6-Cl₂, 4-Br | O |
| 66 | 2-CH₃ | O |
| 67 | 3-CH₃ | O |
| 68 | 4-CH₃ | O |
| 69 | 2,3-(CH₃)₂ | O |
| 70 | 2,4-(CH₃)₂ | O |
| 71 | 2,5-(CH₃)₂ | O |
| 72 | 2,6-(CH₃)₂ | O |
| 73 | 3,4-(CH₃)₂ | O |
| 74 | 3,5-(CH₃)₂ | O |
| 75 | 2,3,5-(CH₃)₃ | O |
| 76 | 2,3,4-(CH₃)₃ | O |
| 77 | 2,3,6-(CH₃)₃ | O |
| 78 | 2,4,5-(CH₃)₃ | O |
| 79 | 2,4,6-(CH₃)₃ | O |
| 80 | 3,4,5-(CH₃)₃ | O |
| 81 | 2,3,4,6-(CH₃)₄ | O |
| 82 | 2,3,5,6-(CH₃)₄ | O |
| 83 | 2,3,4,5,6-(CH₃)₅ | O |
| 84 | 2-C₂H₅ | O |
| 85 | 3-C₂H₅ | O |
| 86 | 4-C₂H₅ | O |
| 87 | 2,4-(C₂H₅)₅ | O |
| 88 | 2,6-(C₂H₅)₂ | O |
| 89 | 3,5-(C₂H₅)₂ | O |
| 90 | 2,4,6-(C₂H₅)₃ | O |
| 91 | 2-n-C₃H₇ | O |
| 92 | 3-n-C₃H₇ | O |
| 93 | 4-n-C₃H₇ | O |
| 94 | 2-i-C₃H₇ | O |
| 95 | 3-i-C₃H₇ | O |
| 96 | 4-i-C₃H₇ | O |
| 97 | 2,4-(i-C₃H₇)₂ | O |
| 98 | 2,6-(i-C₃H₇)₂ | O |
| 99 | 3,5-(i-C₃H₇)₂ | O |
| 100 | 2-s-C₄H₉ | O |
| 101 | 3-s-C₄H₉ | O |
| 102 | 4-s-C₄H₉ | O |
| 103 | 2-t-C₄H₉ | O |
| 104 | 3-t-C₄H₉ | O |
| 105 | 4-t-C₄H₉ | O |
| 106 | 4-n-C₉H₁₉ | O |
| 107 | 2-CH₃, 4-t-C₄H₉ | O |
| 108 | 2-CH₃, 6-t-C₄H₉ | O |
| 109 | 2-CH₃, 4-i-C₃H₇ | O |
| 110 | 2-CH₃, 5-i-C₃H₇ | O |
| 111 | 3-CH₃, 4-i-C₃H₇ | O |
| 112 | 2-cyclo-C₆H₁₁ | O |
| 113 | 3-cyclo-C₆H₁₁ | O |
| 114 | 4-cyclo-C₆H₁₁ | O |
| 115 | 2-C1, 4-C₆H₅ | O |
| 116 | 2-Br, 4-C₆H₅ | O |
| 117 | 2-OCH₃ | O |
| 118 | 3-OCH₃ | O |
| 119 | 4-OCH₃ | O |
| 120 | 2-OC₂H₅ | O |
| 121 | 3-O-C₂H₅ | O |
| 122 | 4-O-C₂H₅ | O |
| 123 | 2-O-n-C₃H₇ | O |
| 124 | 3-O-n-C₃H₇ | O |
| 125 | 4-O-n-C₃H₇ | O |
| 126 | 2-O-i-C₃H₇ | O |
| 127 | 3-O-i-C₃H₇ | O |
| 128 | 4-O-i-C₃H₇ | O |
| 129 | 2-O-n-C₆H₁₃ | O |
| 130 | 3-O-n-C₆H₁₃ | O |
| 131 | 4-O-n-C₆H₁₃ | O |
| 132 | 2-O-CH₂C₆H₅ | O |
| 133 | 3-O-CH₂C₆H₅ | O |
| 134 | 4-O-CH₂C₆ H₅ | O |
| 135 | 2-O-(CH₂)₃C₆H₅ | O |
| 136 | 4-O-(CH₂)₃C₆H₅ | O |
| 137 | 2,3-(OCH₃)₂ | O |
| 138 | 2,4-(OCH₃)₂ | O |
| 139 | 2,5-(OCH₃)₂ | O |
| 140 | 2,6-(OCH₃)₂ | O |
| 141 | 3,4-(OCH₃)₂ | O |
| 142 | 3,5-(OCH₃)₂ | O |
| 143 | 2-O-t-C₄H₉ | O |
| 144 | 3-O-t-C₄H₉ | O |
| 145 | 4-O-t-C₄H₉ | O |
| 146 | 3-(3'-Cl-C₆H₄) | O |
| 147 | 4-(4'-CH₃-C₆H₄) | O |
| 148 | 2-O-C₆H₅ | O |
| 149 | 3-O-C₆H₅ | O |
| 150 | 4-O-C₆H₅ | O |
| 151 | 2-O-(2'-F-C₆H₄) | O |
| 152 | 3-O-(3'-Cl-C₆H₄) | O |
| 153 | 4-O-(4'-CH₃-C₆H₄) | O |
| 154 | 2,3,6-(CH₃)₃, 4-F | O |
| 155 | 2,3,6-(CH₃)₃, 4-Cl | O |
| 156 | 2,3,6-(CH ₃)₃, 4-Br | O |
| 157 | 2,4-(CH₃)₂, 6-F | O |
| 158 | 2,4-(CH₃)₂, 6-Cl | O |
| 159 | 2,4-(CH₃)₂, 6-Br | O |
| 160 | 2-i-C₃H₇, 4-Cl, 5-CH₃ | O |
| 161 | 2-Cl, 4-NO₂ | O |
| 162 | 2-NO₂, 4-Cl | O |
| 163 | 2-OCH₃, 5-NO₂ | O |
| 164 | 2,4-Cl₂, 5-NO₂ | O |
| 165 | 2,4-Cl₂, 6-NO₂ | O |
| 166 | 2,6-Cl₂, 4-NO₂ | O |
| 167 | 2,6-Br₂, 4-NO₂ | O |
| 168 | 2,6-J₂, 4-NO₂ | O |
| 169 | 2-CH₃, 5-i-C₃H₇, 4-Cl | O |
| 170 | 2-CO₂CH₃ | O |
| 171 | 3-CO₂CH₃ | O |
| 172 | 4-CO₂CH₃ | O |
| 173 | 2-CH₂-OCH₃ | O |
| 174 | 3-CH₂-OCH₃ | O |
| 175 | 4-CH₂-OCH₃ | O |
| 176 | 2-Me-4-CH₃-CH(CH₃)-CO | O |
| 177 | 2-CH₃-4-(CH₃-C=NOCH₃) | O |
| 178 | 2-CH₃-4-(CH₃-C=NOC₂H₅) | O |
| 179 | 2-CH₃-4-(CH₃-C=NO-n-C₃H₇) | O |
| 180 | 2-CH₃-4-(CH₃-C=NO-i-C₃H₇) | O |
| 181 | 2,5-(CH₃)₂-4-(CH₃-C=NOCH₃) | O |
| 182 | 2,5-(CH₃)₂-4-(CH₃-C=NOC₂H₅) | O |
| 183 | 2,5-(CH₃-4-(CH₃-C=NO-n-C₃H₇) | O |
| 184 | 2,5-(CH₃)₂-4-(CH₃-C=NO-i-C₃H₇) | O |
| 185 | 2-C₆H₅ | O |
| 186 | 3-C₆H₅ | O |
| 187 | 4-C₆H₅ | O |
| 188 | 2-(2'-F-C₆H₄) | O |
| 189 | 2-CH₃, 5-Br | O |
| 190 | 2-CH₃, 6-Br | O |
| 191 | 2-Cl, 3-CH₃ | O |
| 192 | 2-Cl, 4-CH₃ | O |
| 193 | 2-Cl, 5-CH₃ | O |
| 194 | 2-F, 3-CH₃ | O |
| 195 | 2-F, 4-CH₃ | O |
| 196 | 2-F, 5-CH₃ | O |
| 197 | 2-Br, 3-CH₃ | O |
| 198 | 2-Br, 4-CH₃ | O |
| 199 | 2-Br, 5-CH₃ | O |
| 200 | 3-CH₃, 4-Cl | O |
| 201 | 3-CH₃, 5-Cl | O |
| 202 | 3-CH₃, 4-F | O |
| 203 | 3-CH₃, 5-F | O |
| 204 | 3-CH₃, 4-Br | O |
| 205 | 3-CH₃, 5-Br | O |
| 206 | 3-F, 4-CH₃ | O |
| 207 | 3-Cl, 4-CH₃ | O |
| 208 | 3-Br, 4-CH₃ | O |
| 209 | 2-Cl, 4,5-(CH₃)₂ | O |
| 210 | 2-Br, 4,5-(CH₃)₂ | O |
| 211 | 2-Cl, 3,5-(CH₃)₂ | O |
| 212 | 2-Br, 3,5-(CH₃)₂ | O |
| 213 | 2,6-Cl₂, 4-CH₃ | O |
| 214 | 2,6-F₂, 4-CH₃ | O |
| 215 | 2,6-Br₂, 4-CH₃ | O |
| 216 | 2,4-Br₂, 6-CH₃ | O |
| 217 | 2,4-F₂, 6-CH₃ | O |
| 218 | 2,4-Br₂, 6-CH₃ | O |
| 219 | 2,6-(CH₃)₂, 4-F | O |
| 220 | 2,6-(CH₃)₂, 4-Cl | O |
| 221 | 2,6-(CH₃)₂, 4-Br | O |
| 222 | 3,5-(CH₃)₂, 4-F | O |
| 223 | 3,5-(CH₃)₂, 4-Cl | O |
| 224 | 3,5-(CH₃)₂, 4-Br | O |
| 225 | 2-CF₃ | O |
| 226 | 3-CF₃ | O |
| 227 | 4-CF₃ | O |
| 228 | 2-OCF₃ | O |
| 229 | 3-OCF₃ | O |
| 230 | 4-OCF₃ | O |
| 231 | 3-OCH₂CHF₂ | O |
| 232 | 2-NO₂ | O |
| 233 | 3-NO₂ | O |
| 234 | 4-NO₂ | O |
| 235 | 2-CN | O |
| 236 | 3-CN | O |
| 237 | 4-CN | O |
| 238 | 2-CH₃, 3-Cl | O |
| 239 | 2-CH₃, 4-Cl | O |
| 240 | 2-CH₃, 5-Cl | O |
| 241 | 2-CH₃, 6-Cl | O |
| 242 | 2-CH₃, 3-F | O |
| 243 | 2-CH₃, 4-F | O |
| 244 | 2-CH₃, 5-F | O |
| 245 | 2-CH₃, 6-F | O |
| 246 | 2-CH₃, 3-Br | O |
| 247 | 2-CH₃, 4-Br | O |
| 248 | 2,5-F₂ | O |
| 249 | 2,6-F₂ | O |
| 250 | 3,4-F₂ | O |
| 251 | 3,5-F₂ | O |
| 252 | H | 1 |
| 253 | 2-F | 1 |
| 254 | 3-F | 1 |
| 255 | 4-F | 1 |
| 256 | 2,4-F₂ | 1 |
| 257 | 2,4,6-F₃ | 1 |
| 258 | 2,3,4,5,6-F₅ | 1 |
| 259 | 2,3-F₂ | 1 |
| 260 | 2-Cl | 1 |
| 261 | 3-Cl | 1 |
| 262 | 4-Cl | 1 |
| 263 | 2,3-Cl₂ | 1 |
| 264 | 2,4-Cl₂ | 1 |
| 265 | 2,5-Cl₂ | 1 |
| 266 | 2,6-Cl₂ | 1 |
| 267 | 3,4-Cl₂ | 1 |
| 268 | 3,5-Cl₂ | 1 |
| 269 | 2,3,4-Cl₃ | 1 |
| 270 | 2,3,5-Cl₃ | 1 |
| 271 | 2,3,6-Cl₃ | 1 |
| 272 | 2,4,5-Cl₃ | 1 |
| 273 | 2,4,6-Cl₃ | 1 |
| 274 | 3,4,5-Cl₃ | 1 |
| 275 | 2,3,4,6-Cl₄ | 1 |
| 276 | 2,3,5,6-Cl₄ | 1 |
| 277 | 2,3,4,5,6-Cl₅ | 1 |
| 278 | 2-Br | 1 |
| 279 | 3-Br | 1 |
| 280 | 4-Br | 1 |
| 281 | 2,4-Br₂ | 1 |
| 282 | 2,5-Br₂ | 1 |
| 283 | 2,6-Br₂ | 1 |
| 284 | 2,4,6-Br₃ | 1 |
| 285 | 2,3,4,5,6-Br₅ | 1 |
| 286 | 2-J | 1 |
| 287 | 3-J | 1 |
| 288 | 4-J | 1 |
| 289 | 2,4-J₂ | 1 |
| 290 | 2-Cl, 3-F | 1 |
| 291 | 2-Cl, 4-F | 1 |
| 292 | 2-Cl, 5-F | 1 |
| 293 | 2-Cl, 6-F | 1 |
| 294 | 2-Cl, 3-Br | 1 |
| 295 | 2-Cl, 4-Br | 1 |
| 296 | 2-Cl, 5-Br | 1 |
| 297 | 2-Cl, 6-Br | 1 |
| 298 | 2-Br, 3-Cl | 1 |
| 299 | 2-Br, 4-Cl | 1 |
| 300 | 2-Br, 5-Cl | 1 |
| 301 | 2-Br, 3-F | 1 |
| 302 | 2-Br, 4-F | 1 |
| 303 | 2-Br, 5-F | 1 |
| 304 | 2-Br, 6-F | 1 |
| 305 | 2-F, 3-Cl | 1 |
| 306 | 2-F, 4-Cl | 1 |
| 307 | 2-F, 5-Cl | 1 |
| 308 | 3-Cl, 4-F | 1 |
| 309 | 3-Cl, 5-F | 1 |
| 310 | 3-Cl, 4-Br | 1 |
| 311 | 3-Cl, 5-Br | 1 |
| 312 | 3-F, 4-Cl | 1 |
| 313 | 3-F, 4-Br | 1 |
| 314 | 3-Br, 4-Cl | 1 |
| 315 | 3-Br, 4-F | 1 |
| 316 | 2,6-Cl₂, 4-Br | 1 |
| 317 | 2-CH₃ | 1 |
| 318 | 3-CH₃ | 1 |
| 319 | 4-CH₃ | 1 |
| 320 | 2,3-(CH₃)₂ | 1 |
| 321 | 2,4-(CH₃)₂ | 1 |
| 322 | 2,5-(CH₃)₂ | 1 |
| 323 | 2,6-(CH₃)₂ | 1 |
| 324 | 3,4-(CH₃)₂ | 1 |
| 325 | 3,5-(CH₃)₂ | 1 |
| 326 | 2,3,5-(CH₃)₃ | 1 |
| 327 | 2,3,4-(CH₃)₃ | 1 |
| 328 | 2,3,6-(CH₃)₃ | 1 |
| 329 | 2,4,5-(CH₃)₃ | 1 |
| 330 | 2,4,6-(CH₃)₃ | 1 |
| 331 | 3,4,5-(CH₃)₃ | 1 |
| 332 | 2,3,4,6-(CH₃)₄ | 1 |
| 333 | 2,3,5,6-(CH₃)₄ | 1 |
| 334 | 2,3,4,5,6-(CH₃)₅ | 1 |
| 335 | 2-C₂H₅ | 1 |
| 336 | 3-C₂ H₅ | 1 |
| 337 | 4-C₂H₅ | 1 |
| 338 | 2,4-(C₂H₅)₅ | 1 |
| 339 | 2,6-(C₂H₅)₂ | 1 |
| 340 | 3,5-(C₂H₅)₂ | 1 |
| 341 | 2,4,6-(C₂H₅)₃ | 1 |
| 342 | 2-n-C₃H₇ | 1 |
| 343 | 3-n-C₃H₇ | 1 |
| 344 | 4-n-C₃H₇ | 1 |
| 345 | 2-i-C₃H₇ | 1 |
| 346 | 3-i-C₃H₇ | 1 |
| 347 | 4-i-C₃H₇ | 1 |
| 348 | 2,4-(i-C₃H₇)₂ | 1 |
| 349 | 2,6-(i-C₃H₇)₂ | 1 |
| 350 | 3,5-(i-C₃H₇)₂ | 1 |
| 351 | 2-s-C₄H₉ | 1 |
| 352 | 3-s-C₄H₉ | 1 |
| 353 | 4-s-C₄H₉ | 1 |
| 354 | 2-t-C₄H₉ | 1 |
| 355 | 3-t-C₄H₉ | 1 |
| 356 | 4-t-C₄H₉ | 1 |
| 357 | 4-n-C₉H₁₉ | 1 |
| 358 | 2-CH₃, 4-t-C₄H₉ | 1 |
| 359 | 2-CH₃, 6-t-C₄H₉ | 1 |
| 360 | 2-CH₃, 4-i-C₃H₇ | 1 |
| 361 | 2-CH₃, 5-i-C₃H₇ | 1 |
| 362 | 3-CH₃, 4-i-C₃H₇ | 1 |
| 363 | 2-cyclo-C₆H₁₁ | 1 |
| 364 | 3-cyclo-C₆H₁₁ | 1 |
| 365 | 4-cyclo-C₆H₁₁ | 1 |
| 366 | 2-Cl, 4-C₆H₅ | 1 |
| 367 | 2-Br, 4-C₆H₅ | 1 |
| 368 | 2-OCH₃ | 1 |
| 369 | 3-OCH₃ | 1 |
| 370 | 4-OCH₃ | 1 |
| 371 | 2-OC₂H₅ | 1 |
| 372 | 3-O-C₂H₅ | 1 |
| 373 | 4-O-C₂H₅ | 1 |
| 374 | 2-O-n-C₃H₇ | 1 |
| 375 | 3-O-n-C₃H₇ | 1 |
| 376 | 4-O-n-C₃H₇ | 1 |
| 377 | 2-O-i-C₃H₇ | 1 |
| 378 | 3-O-i-C₃H₇ | 1 |
| 379 | 4-O-i-C₃H₇ | 1 |
| 380 | 2-O-n-C₆H₁₃ | 1 |
| 381 | 3-O-n-C₆H₁₃ | 1 |
| 382 | 4-O-n-C₆H₁₃ | 1 |
| 383 | 2-O-CH₂C₆ H₅ | 1 |
| 384 | 3-O-CH₂C₆ H₅ | 1 |
| 385 | 4-O-CH₂C₆H₅ | 1 |
| 386 | 2-O-(CH₂)₃ C₆H₅ | 1 |
| 387 | 4-O-(CH₂)₃ C₆H₅ | 1 |
| 388 | 2,3-(OCH₃)₂ | 1 |
| 389 | 2,4-(OCH₃)₂ | 1 |
| 390 | 2,5-(OCH₃)₂ | 1 |
| 391 | 2,6-(OCH₃)₂ | 1 |
| 392 | 3,4-(OCH₃)₂ | 1 |
| 393 | 3,5-(OCH₃)₂ | 1 |
| 394 | 2-O-t-C₄H₉ | 1 |
| 395 | 3-O-t-C₄H₉ | 1 |
| 396 | 4-O-t-C₄H₉ | 1 |
| 397 | 3-(3'-Cl-C₆H₄) | 1 |
| 398 | 4-(4'-CH₃-C₆H₄) | 1 |
| 399 | 2-O-C₆H₅ | 1 |
| 400 | 3-O-C₆H₅ | 1 |
| 401 | 4-O-C₆H₅ | 1 |
| 402 | 2-O-(2'-F-C₆H₄) | 1 |
| 403 | 3-O-(3'-Cl-C₆H₄) | 1 |
| 404 | 4-O-(4'-CH₃-C₆H₄) | 1 |
| 405 | 2,3,6-(CH₃)₃, 4-F | 1 |
| 406 | 2,3,6-(CH₃)₃, 4-Cl | 1 |
| 407 | 2,3,6-(CH₃)₃, 4-Br | 1 |
| 408 | 2,4-(CH₃)₂, 6-F | 1 |
| 409 | 2,4-(CH₃)₂, 6-Cl | 1 |
| 410 | 2,4-(CH₃)₂, 6-Br | 1 |
| 411 | 2-i-C₃H₇, 4-Cl, 5-CH₃ | 1 |
| 412 | 2-Cl, 4-NO₂ | 1 |
| 413 | 2-NO₂, 4-Cl | 1 |
| 414 | 2-OCH₃, 5-NO₂ | 1 |
| 415 | 2,4-Cl₂, 5-NO₂ | 1 |
| 416 | 2,4-Cl₂, 6-NO₂ | 1 |
| 417 | 2,6-Cl₂, 4-NO₂ | 1 |
| 418 | 2,6-Br₂, 4-NO₂ | 1 |
| 419 | 2,6-J₂, 4-NO₂ | 1 |
| 420 | 2-CH₃, 5-i-C₃H₇, 4-Cl | 1 |
| 421 | 2-CO₂CH₃ | 1 |
| 422 | 3-CO₂CH₃ | 1 |
| 423 | 4-CO₂CH₃ | 1 |
| 424 | 2-CH₂-OCH₃ | 1 |
| 425 | 3-CH₂-OCH₃ | 1 |
| 426 | 4-CH₂-OCH₃ | 1 |
| 427 | 2-Me-4-CH₃-CH(CH₃)-CO | 1 |
| 428 | 2-CH₃-4-(CH₃-C=NOCH₃) | 1 |
| 429 | 2-CH₃-4-(CH₃-C=NOC₂H₅) | 1 |
| 430 | 2-CH₃-4-(CH₃-C=NO-n-C₃H₇) | 1 |
| 431 | 2-CH₃-4-(CH₃-C=NO-i-C₃H₇) | 1 |
| 432 | 2,5-(CH₃)₂-4-(CH₃-C=NOCH₃) | 1 |
| 433 | 2,5-(CH₃)₂-4-(CH₃-C=NOC₂H₅) | 1 |
| 434 | 2,5-(CH₃-4-(CH₃-C=NO-n-C₃H₇) | 1 |
| 435 | 2,5-(CH₃)₂-4-(CH₃-C=NO-i-C₃H₇) | 1 |
| 436 | 2-C₆H₅ | 1 |
| 437 | 3-C₆H₅ | 1 |
| 438 | 4-C₆H₅ | 1 |
| 439 | 2-(2'-F-C₆H₄) | 1 |
| 440 | 2-CH₃, 5-Br | 1 |
| 441 | 2-CH₃, 6-Br | 1 |
| 442 | 2-Cl, 3-CH₃ | 1 |
| 443 | 2-Cl, 4-CH₃ | 1 |
| 444 | 2-Cl, 5-CH₃ | 1 |
| 445 | 2-F, 3-CH₃ | 1 |
| 446 | 2-F, 4-CH₃ | 1 |
| 447 | 2-F, 5-CH₃ | 1 |
| 448 | 2-Br, 3-CH₃ | 1 |
| 449 | 2-Br, 4-CH₃ | 1 |
| 450 | 2-Br, 5-CH₃ | 1 |
| 451 | 3-CH₃, 4-Cl | 1 |
| 452 | 3-CH₃, 5-Cl | 1 |
| 453 | 3-CH₃, 4-F | 1 |
| 454 | 3-CH₃, 5-F | 1 |
| 455 | 3-CH₃, 4-Br | 1 |
| 456 | 3-CH₃, 5-Br | 1 |
| 457 | 3-F, 4-CH₃ | 1 |
| 458 | 3-Cl, 4-CH₃ | 1 |
| 459 | 3-Br, 4-CH₃ | 1 |
| 460 | 2-Cl, 4,5-(CH₃)₂ | 1 |
| 461 | 2-Br, 4,5-(CH₃)₂ | 1 |
| 462 | 2-Cl, 3,5-(CH₃)₂ | 1 |
| 463 | 2-Br, 3,5-(CH₃)₂ | 1 |
| 464 | 2,6-Cl₂, 4-CH₃ | 1 |
| 465 | 2,6-F₂, 4-CH₃ | 1 |
| 466 | 2,6-Br₂ , 4-CH₃ | 1 |
| 467 | 2,4-Br₂, 6-CH₃ | 1 |
| 468 | 2,4-F₂, 6-CH₃ | 1 |
| 469 | 2,4-Br₂, 6-CH₃ | 1 |
| 470 | 2,6-(CH₃)₂, 4-F | 1 |
| 471 | 2,6-(CH₃)₂, 4-Cl | 1 |
| 472 | 2,6-(CH₃)₂, 4-Br | 1 |
| 473 | 3,5-(CH₃)₂, 4-F | 1 |
| 474 | 3,5-(CH₃)₂, 4-Cl | 1 |
| 475 | 3,5-(CH₃)₂, 4-Br | 1 |
| 476 | 2-CF₃ | 1 |
| 477 | 3-CF₃ | 1 |
| 478 | 4-CF₃ | 1 |
| 479 | 2-OCF₃ | 1 |
| 480 | 3-OCF₃ | 1 |
| 481 | 4-OCF₃ | 1 |
| 482 | 3-OCH₂CHF₂ | 1 |
| 483 | 2-NO₂ | 1 |
| 484 | 3-NO₂ | 1 |
| 485 | 4-NO₂ | 1 |
| 486 | 2-CN | 1 |
| 487 | 3-CN | 1 |
| 488 | 4-CN | 1 |
| 489 | 2-CH₃, 3-Cl | 1 |
| 490 | 2-CH₃, 4-Cl | 1 |
| 491 | 2-CH₃, 5-Cl | 1 |
| 492 | 2-CH₃, 6-Cl | 1 |
| 493 | 2-CH₃, 3-F | 1 |
| 494 | 2-CH₃, 4-F | 1 |
| 495 | 2-CH₃, 5-F | 1 |
| 496 | 2-CH₃, 6-F | 1 |
| 497 | 2-CH₃, 3-Br | 1 |
| 498 | 2-CH₃, 4-Br | 1 |
| 499 | 2,5-F₂ | 1 |
| 500 | 2,6-F₂ | 1 |
| 501 | 3,4-F₂ | 1 |
| 502 | 3,5-F₂ | 1 |

Die erfindungsgemäßen Verbindungen der Formel I eignen sich zur Bekämpfung von Schadpilzen und von tierischen Schädlingen aus der Klasse der Insekten, Spinnentiere und Nematoden. Sie können im Pflanzenschutz sowie auf dem Hygiene-, Vorratsschutz- und Veterinärsektor als Fungizide und Schädlingsbekämpfungsmittel eingesetzt werden.

Zu den schädlichen Insekten gehören:
aus der Ordnung der Schmetterlinge (Lepidoptera) beispielsweise Adoxophyes orana, Agrotis ypsilon, Agrotis segetum, Alabama argillacea, Anticarsia gemmatalis, Argyresthia conjugella, Autographa gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha, Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis, Thaumatopoea pityocampa, Tortrix viridana, Trichoplusia ni, Tryporyza incertulas, Zeiraphera canadensis,
ferner Galleria mellonella und Sitotroga cerealella, Ephestia cautella, Tineola bisselliella;
aus der Ordnung der Käfer (Coleoptera) beispielsweise Agriotes lineatus, Agriotes obscurus, Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllopertha horticola, Phyllophaga sp., Phyllotreta chrysocephala, Phyllotreta nemorum, Phyllotreta striolata, Popillia japonica, Psylliodes napi, Scolytus intricatus, Sitona lineatus,
ferner Bruchus rufimanus, Bruchus pisorum, Bruchus lentis, Sitophilus granaria, Lasioderma serricorne, Oryzaephilus surinamensis, Rhyzopertha dominica, Sitophilus oryzae, Tribolium castaneum, Trogoderma granarium, Zabrotes subfasciatus;
aus der Ordnung der Zweiflügler (Diptera) beispielsweise Anastrepha ludens, Ceratitis capitata, Contarinia sorghicola, Dacus cucurbitae, Dacus oleae, Dasineura brassicae, Delia coarctata, Delia radicum, Hydrellia griseola, Hylemyia platura, Liriomyza sativae, Liriomyza trifolii, Mayetiola destructor, Orseolia oryzae, Oscinella frit, Pegomya hyoscyami, Phorbia antiqua, Phorbia brassicae, Phorbia coarctata, Rhagoletis cerasi, Rhagoletis pomonella, Tipula oleracea, Tipula paludosa,
ferner Aedes aegypti, Aedes vexans, Anopheles maculipennis, Chrysomya bezziana, Chrysomya hominivorax, Chrysomya macellaria, Cordylobia anthropophaga, Culex pipiens, Fannia canicularis, Gasterophilus intestinalis, Glossina morsitans, Haematobia irritans, Haplodiplosis equestris, Hypoderma lineata, Lucilia caprina, Lucilia cuprina, Lucilia sericata, Musca domestica, Muscina stabulans, Oestrus ovis, Tabanus bovinus, Simulium damnosum;
aus der Ordnung der Thripse (Thysanoptera) beispielsweise Frankliniella fusca, Frankliniella occidentalis, Frankliniella tritici, Haplothrips tritici, Scirtothrips citri, Thrips oryzae, Thrips palmi, Thrips tabaci;
aus der Ordnung der Hautflügler (Hymenoptera) beispielsweise Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri;
aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus hesperus, Lygus lineolaris, Lygus pratensis, Nezara viridula, Piesma quadrata, Solubea insularis, Thyanta perditor;
aus der Ordnung der Pflanzensauger (Homoptera) beispielsweise Acyrthosiphon onobrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Planococcus citri, Psylla mali, Psylla piri, Psylla pyricol, Quadraspidiotus perniciosus, Rhopalosiphum maidis, Saissetia oleae, Schizaphis graminum, Selenaspidus articulatus, Sitobion avenae, Sogatella furcifera, Toxoptera citricida, Trialeurodes abutilonea, Trialeurodes vaporariorum, Viteus vitifolii;
aus der Ordnung der Termiten (Isoptera) beispielsweise Calotermes flavicollis, Leucotermes flavipes, Macrotermes subhyalinus, Odontotermes formosanus, Reticulitermes lucifugus, Termes natalensis;
aus der Ordnung der Geradflügler (Orthoptera) beispielsweise Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria,
ferner Acheta domestica, Blatta orientalis, Blattella germanica, Periplaneta americana;
aus der Ordnung der Arachnoidea beispielsweise phytophage Milben wie Aculops lycopersicae, Aculops pelekassi, Aculus schlechtendali, Brevipalpus phoenicis, Bryobia praetiosa, Eotetranychus carpini, Eutetranychus banksii, Eriophyes sheldoni, Oligonychus pratensis, Panonychus ulmi, Panonychus citri, Phyllocoptruta oleivora, Polyphagotarsonemus latus, Tarsonemus pallidus, Tetranychus cinnabarinus, Tetranychus kanzawai, Tetranchus pacificus, Tetranychus urticae, Zecken wie Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Rhipicephalus appendiculatus und Rhipicephalus evertsi sowie tierparasitische Milben wie Dermanyssus gallinae, Psoroptes ovis und Sarcoptes scabiei;
aus der Klasse der Nematoden beispielsweise Wurzelgallennematoden, z.B. Meloidogyne hapla, Meloidogyne incognita, Meloidogyne javanica, zystenbildende Nematoden, z.B. Globodera pallida, Globodera rostochiensis, Heterodera avenae, Heterodera glycines, Heterodera schachtii, migratorische Endoparasiten und semi-endoparasitische Nematoden, z.B. Heliocotylenchus multicinctus, Hirschmanniella oryzae, Hoplolaimus spp, Pratylenchus brachyurus, Pratylenchus fallax, Pratylenchus penetrans, Pratylenchus vulnus, Radopholus similis, Rotylenchus reniformis, Scutellonema bradys, Tylenchulus semipenetrans, Stock- und Blattnematoden z.B. Anguina tritici, Aphelenchoides besseyi, Ditylenchus angustus, Ditylenchus dipsaci, Virusvektoren, z.B. Longidorus spp, Trichodorus christei, Trichodorus viruliferus, Xiphinema index, Xiphinema mediterraneum.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Als Fungizide sind die α-Phenylbutensäuremethylester der Formel I z.T. systemisch wirksam. Sie können als Blatt- und Bodenfungizide gegen ein breites Spektrum von pflanzenpathogenen Pilzen, insbesondere aus der Klasse der Ascomyceten, Deuteromyceten, Phycomyceten und Basidiomyceten eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zukkerrohr, Wein, Obst- und Zierpflanzen und Gemüsepflanzen wie Gurken, Bohnen und Kürbisgewächsen, sowie an den Samen dieser Pflanzen.

Speziell eignen sich die Verbindungen I zur Bekämpfung folgender Pflanzenkrankheiten:
* Erysiphe graminis (echter Mehltau) in Getreide,
* Erysiphe cichoracearum und Sphaerotheca fuliginea an Kürbisgewächsen,
* Podosphaera leucotricha an Äpfeln,
* Uncinula necator an Reben,
* Puccinia-Arten an Getreide,
* Rhizoctonia-Arten an Baumwolle und Rasen,
* Ustilago-Arten an Getreide und Zuckerrohr,
* Venturia inaequalis (Schorf) an Äpfeln,
* Helminthosporium-Arten an Getreide,
* Septoria nodorum an Weizen,
* Botrytis cinerea (Grauschimmel) an Erdbeeren, Reben,
* Cercospora arachidicola an Erdnüssen,
* Pseudocercosporella herpotrichoides an Weizen, Gerste,
* Pyricularia oryzae an Reis,
* Phytophthora infestans an Kartoffeln und Tomaten,
* Fusarium- und Verticillium-Arten an verschiedenen Pflanzen,
* Plasmopara viticola an Reben,
* Alternaria-Arten an Gemüse und Obst.

Die neuen Verbindungen können auch im Materialschutz (Holzschutz) eingesetzt werden, z.B. gegen Paecilomyces variotii.

Sie können in die üblichen Formulierungen übergeführt werden, wie Lösungen, Emulsionen, Suspensionen, Stäube, Pulver, Pasten oder Granulate. Die Anwendungformen richten sich dabei nach dem jeweiligen Verwendungszweck; sie sollten in jedem Fall möglichst die feinste Verteilung der Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht:
- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;
- Trägerstoffe wie natürliche Gesteinsmehle (z.B. Kaoline, Ton-erden, Talkum, Kreide) und synthetische Gesteinsmehle (z.B. hochdisperse Kieselsäure, Silikate);
- Emulgiermittel wie nichtionogene und anionische Emulgatoren (z.B. Polyoxyethylen-Fettalkohol-Ether, Alkylsulfonate und Arylsulfonate) und
- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Wirkstoffkonzentrationen in den anwendungsfertigen Zubereitungen können in größeren Bereichen variiert werden.

Ganz allgemein enthalten die Mittel zwischen 0,0001 und 95 Gew.-% Wirkstoff.

Formulierungen mit mehr als 95 Gew.-% Wirkstoff können mit gutem Erfolg im Ultra-Low-VolumeVerfahren (ULV) ausgebracht werden, wobei sogar der Wirksoff ohne Zusätze verwendet werden kann.

Für die Anwendung als Fungizide empfehlen sich Konzentrationen zwischen 0,01 und 95 Gew.%, vorzugsweise zwischen 0,5 und 90 Gew.%, Wirkstoff. Für die Anwendung als Insektizide kommen Formulierungen mit 0,0001 bis 10 Gew.%, vorzugsweise 0,01 bis 1 Gew.% Wirkstoff, in Betracht.

Die Wirkstoffe werden normalerweise in einer Reinheit von 90 % bis 100 %, vorzugsweise 95 % bis 100 % (nach NMR-Spektrum) eingesetzt.

Beispiele für solche Zubereitungen sind:
I. eine Lösung aus 90 Gew.-Teilen einer erfindungsgemäßen Verbindung I und 10 Gew.-Teilen N-Methyl-α-pyrrolidon, die zur Anwendung in Form kleinster Tropfen geeignet ist;
II. eine Lösung von 20 Gew.-Teilen einer erfindungsgemäßen 0 Verbindung I in einer Mischung aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylen oxid an 1 Mol Ricinusöl; durch feines Verteilen der Formulierung in Wasser erhält man eine Dispersion.
III. eine Lösung von 20 Gew.-Teilen einer erfindungsgemäßen Verbindung I in einer Mischung aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl; durch feines Verteilen der Formulierung in Wasser erhält man eine Dispersion.
IV. eine wäßrige Dispersion aus 20 Gew.-Teilen einer erfindungsgemäßen Verbindung I, in einer Mischung aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl; durch feines Verteilen der Formulierung in Wasser erhält man eine Dispersion.
V. eine in einer Hammermühle vermahlene Mischung aus 20 Gew.-Teilen einer erfindungsgemäßen Verbindung I, 3 Gew.-Teilen des Natriumsalzes der Diisobutylnaphtalin-α-sulfonsäure, 17 Gew.-Teilen des Natriumsalzes einer Ligninsulfonsäure aus einer Sulfitablauge und 60 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 92 Gew.-Teilen pulverförmigem Kieselsäuregel und 8 Gew.Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkohol-polyglykol-ether, 2 Gew.-Teilen des Natriumsalzes eines Phenolsulfonsäure-harnstoff-formaldehyd-Kondensates und 68 Gew.-Teilen eines paraffinischen Mineralöls;
X. eine in einer Hammermühle vermahlene Mischung aus 10 Gew.-Teilen einer erfindungsgemäßen Verbindung I, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Verbindungen I werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die Aufwandmengen liegen je nach Art des gewünschten Effektes zwischen 0,02 und 3 kg Wirkstoff pro ha, vorzugsweise bei 0,1 bis 1 kg/ha.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Schädlingen beträgt unter Freilandbedingungen 0,02 bis 10, vorzugsweise 0,1 bis 2,0 kg/ha Wirkstoff.

Die Verbindungen I, allein oder in Kombination mit Herbiziden oder Fungiziden, können auch noch mit weiteren Pflanzenschutzmitteln gemischt gemeinsam ausgebracht werden, beispielsweise mit Wachstumsregulatoren oder mit Mitteln zur Bekämpfung von Schädlingen oder Bakterien. Von Interesse ist ferner die Mischbarkeit mit Düngemitteln oder mit Mineralsalzlösungen, welche zur Behebung von Ernährungs- und Spurenelementmängeln eingesetzt werden.

Die Pflanzenschutz- und Düngemittel können zu den erfindungsgemäßen Mitteln im Gewichtsverhältnis 1:10 bis 10:1 zugesetzt werden, gegebenenfalls auch erst unmittelbar vor der Anwendung (Tankmix). Beim Vermischen mit Fungiziden oder Insektiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid; Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthals°ure-di-isopropylester;
heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-β-[bis-(dimethylamino)-phosphinyl]-3-phenyl-1,2,4-triazol, 2,3-Dicyano-1,4-dithioanthrachinon, 2-Thio-1,3-dithiolo-β-[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid, N-Dichlorfluormethylthio-N',N'dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethylfuran-3-carbonsäurecyclohexylamid, N-Cyclohexyl-N-methoxy-2,5-dimethyl-furan-3-carbonsäureamid, 2-Methyl-benzoesäureanilid, 2-Iod-benzoesäure-anilid, N-Formyl-N-morpholin-2,2,2-trichlorethylacetal, Piperazin-1,4-diylbis-(1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclodedecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(pchlorphenyl)-3-pyridinmethanol, 1,2-Bis-(3-ethoxycarbonyl-2-thioureido)-benzol, 1,2-Bis-(3-methoxycarbonyl-2-thioureido) -benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-a-(lH-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Daten aufgeführt.

### 1. α-(2-(N'-(o-Chlorphenyl)-triazolyl-3'-oxymethyl)-phenyl)- β-methoxy-acrylsäuremethylester

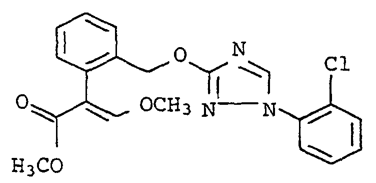

Eine Mischung von 4 g (14 mmol) α-(o-Brommethylphenyl-β-methoxy-acrylsäuremethylester (EP 203 606), 2,7 g (14 mmol)N-(o-Chlorphenyl)-3-hydroxitriazol und 2,9 g (20 mmol) K₂CO₃ in 10 ml Dimethylformamid wurde über Nacht bei Raumtemperatur (= 25°C) gerührt. Anschließend wurde die Reaktionsmischung mit Wasser verdünnt und dreimal mit Methyl-t-butylether extrahiert. Die vereinigten organischen Phasen wurden mit Wasser extrahiert, über MgSO₄ getrocknet und eingeengt. Der Rückstand wurde säulenchromatographisch mit Cyclohexan/Essigester-Gemischen gereinigt. Das so erhaltene Produkt kristallisierte und wurde mit Methyl-t-butylether ausgerührt. Man erhielt 0,48 g (12 %) der Titelverbindung als farblosen Festkörper (Fp = 120°C).
¹H-NMR (CDCl₃; δ in ppm):
8,25 (s, 1H, Triazolyl); 7,1-7,8 (9H, Phenyl, Vinyl); 5,3 (s, 2H, OCH₂); 3,8; 3,7 (2s, je 3H, 2 x OCH₃)

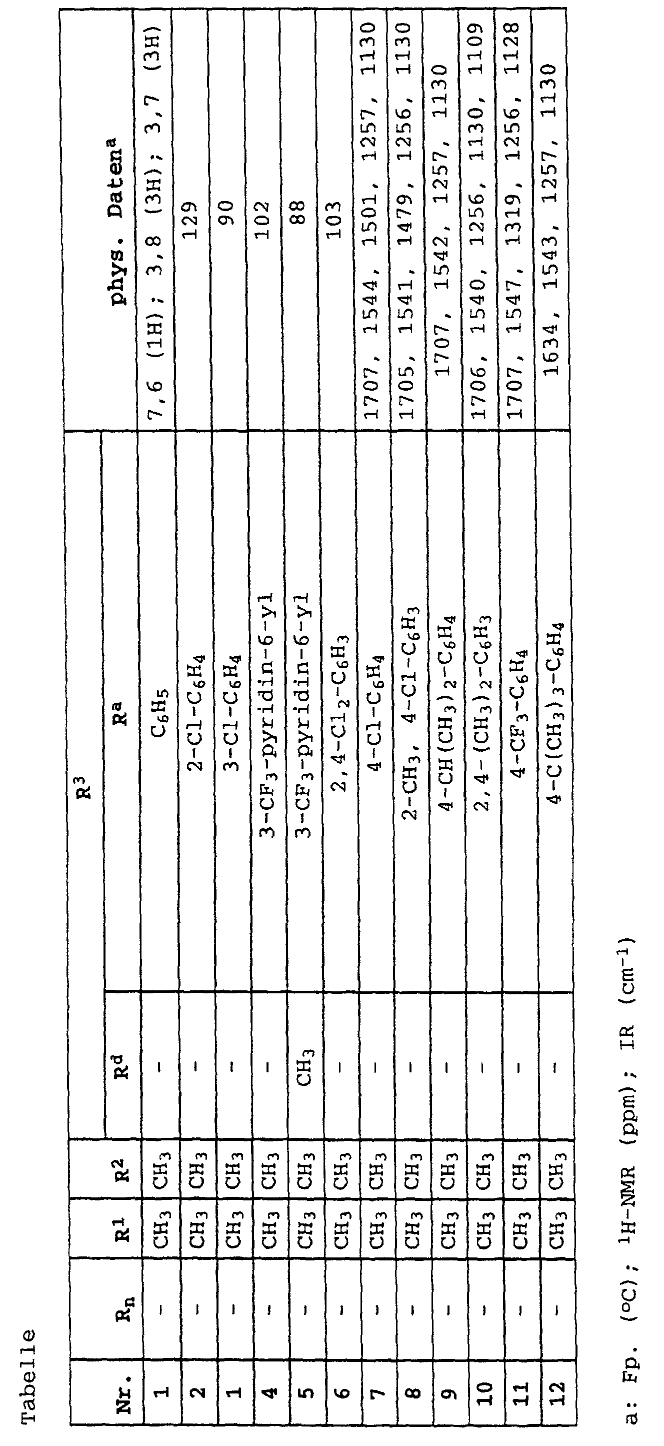

### Beispiele zur Wirkung gegen Schadpilze

Die fungizide Wirkung der Verbindungen der Formel I ließ sich durch folgende Versuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Wirkung gegen Plasmopara viticola (Rebenperonospora)

Topfreben (Sorte: "Müller Thurgau") wurden mit der Wirkstoffaufbereitung tropfnaß gespritzt (Aufwandmenge: 16 ppm). Nach 8 Tagen wurden die Pflanzen mit einer Zoosporenaufschwemmung des Pilzes *Plasmopara viticola* besprüht und 5 Tage bei 20-30°C bei hoher Luftfeuchtigkeit bewahrt. Vor der Beurteilung wurden die Pflanzen danach für 16 h bei hoher Luftfeuchtigkeit bewahrt. Die Auswertung erfolgte visuell.

In diesem Test zeigten die mit den erfindungsgemäßen Verbindungen Nrn. 1-3 und 6-12 behandelten Pflanzen einen Befall von 15% und weniger während die unbehandelten (Kontroll-) Pflanzen zu 70% befallen waren.

### Wirkung gegen Puccinia recondita (Weizenbraunrost)

Blätter von Weizensämlingen (Sorte "Kanzler") wurden mit Sporen des Braunrosts (*Puccinia recondita*) bestäubt. Die so behandelten Pflanzen wurden 24 h bei 20-22°C und einer relativen Luftfeuchtigkeit von 90-95% inkubiert und anschließend mit der wäßrigen Wirkstoffaufbereitung behandelt (Aufwandmenge: 63 ppm). Nach weiteren 8 Tagen bei 20-22°C und 65-70% relativer Luftfeuchtigkeit wurde das Ausmaß der Pilzentwicklung ermittlelt. Die Auswertung erfolgte visuell.

In diesem Test zeigten die mit den erfindungsgemäßen Verbindungen Nrn. 1 und 3-12 behandelten Pflanzen einen Befall von 15% und weniger während die unbehandelten (Kontroll-) Pflanzen zu 65% befallen waren.

### Wirkung gegen Pyricularia oryzae (Reisbrand)

Reiskeimlinge (Sorte: "Tai Nong 67") wurden mit der Wirkstoffaufbereitung tropfnaß gespritzt (Aufwandmenge: 63 ppm). Nach 24 Stunden wurden die Pflanzen mit einer wäßrigen Sporensuspension des Pilzes *Pyricularia oryzae* besprüht und 6 Tage bei 22-24°C bei einer relativen Luftfeuchtigkeit von 95-99% bewahrt. Die Beurteilung erfolgte visuell.

In diesem Test zeigten die mit den erfindungsgemäßen Verbindungen Nrn. 1 und 3-6 behandelten Pflanzen einen Befall von 15% und weniger während die unbehandelten (Kontroll-) Pflanzen zu 85% befallen waren.

In einem entsprechenden Test zeigten die mit den erfindungsgemäßen Verbindungen Nrn. 7-9 behandelten Pflanzen einen Befall von 15% und weniger während die unbehandelten (Kontroll-) Pflanzen zu 65% befallen waren.

### Beispiele zur Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

### Nephotettix cincticeps (Grüne Reiszikade), Kontaktwirkung

Rundfilter wurden mit der wäßrigen Wirkstoffaufbereitung behandelt und anschließend mit 5 adulten Zikaden belegt. Nach 24h wurde die Mortalität beurteilt.

In diesem Test zeigte die Verbindung 11 eine Wirkschwelle von 0,4 mg.

### Tetranychus telarius (Rote Spinne), Kontaktwirkung

Stark befallene getopfte Buschbohnen, die das zweite Folgeblattpaar zeigten, wurden mit wässrigen Wirkstoffaufbereitung behandelt. Nach 5 Tagen im Gewächshaus wurde der Bekämpfungserfolg mittels Binokular bestimmt.

In diesem Test zeigte die Verbindung 8 eine Wirkschwelle von 400 ppm.

## Patentansprüche

1. Verbindungen der allgemeinen Formel I 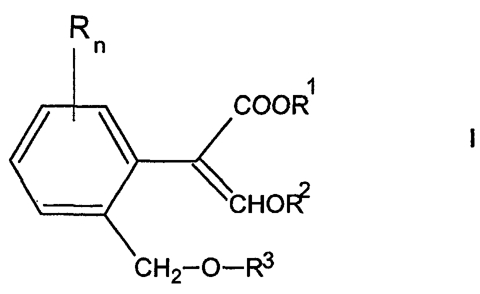 in der der Index und die Substituenten die folgende Bedeutung haben:
n 0, 1, 2, 3 oder 4, wobei die Substituenten R verschieden sein können, wenn n größer als 1 ist;
R Nitro, Cyano, Halogen,
C₁-C₁₀-Alkyl, C₂-C₁₀-Alkenyl, C₂-C₁₀-Alkinyl, C₁-C₁₀-Alkoxy, C₂-C₁₀-Alkenyloxy, C₂-C₁₀-Alkinyloxy
wobei die aliphatischen Gruppen partiell oder vollständig halogeniert sein können und/oder einen bis drei, insbesondere einen, der folgenden Reste tragen können:
Nitro, Cyano, C₁-C₄-Alkoxy, C₁-C₄-Alkoxycarbonyl oder eine Gruppe =N-OR^{x}, in der R^{x} für C₁-C₆-Alkyl, C₃-C₆-Alkenyl und C₃-C₆-Alkinyl steht,
oder ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann,
welches wiederum partiell oder vollständig halogeniert sein kann und/oder einen bis drei, der folgenden Reste tragen kann: Nitro, Cyano, C₁-C₄-Alkyl, C₁-C₄-Alkoxy und C₁-C₄-Alkoxycarbonyl;
R¹,R² C₁-C-Alkyl;
R³ ein Triazolrest der Formel A 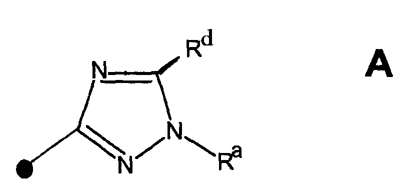
wobei die mit • gekennzeichnete Bindung der Bindung zum Sauerstoff entspricht und in denen die Substituenten die folgende Bedeutung haben:
R^{a} C₁-C₁₀-Alkyl, C₂-C₁₀-Alkenyl oder C₂-C₁₀-Alkinyl; wobei die aliphatischen Gruppen und/oder einen bis drei, insbesondere einen, der folgenden Reste tragen können:
Nitro, Cyano, C₁-C₄-Alkoxy, C₁-C₄-Alkoxycarbonyl oder eine Gruppe =N-OR^{x}, in der R^{x} für C₁-C₆-Alkyl, C₃-C₆-Alkenyl und C₃-C₆-Alkinyl steht,
oder ein ein- oder zweikerniges aromatisches Ringsystem, welches neben Kohlenstoffatomen ein bis vier Stickstoffatome oder ein oder zwei Stickstoffatome und ein oder zwei Sauerstoff- oder Schwefelatome oder ein Sauerstoff- oder Schwefelatom als Ringglieder enthalten kann,
welches wiederum partiell oder vollständig halogeniert sein kann und/oder einen bis drei der folgenden Reste tragen kann: Nitro, Cyano, C₁-C₄-Alkyl, C₁-C₄-Alkoxy und C₁-C₄-Alkoxycarbonyl;
R^{d} Wasserstoff, Cyano, Halogen,
C₁-C₁₀-Alkyl, C₁-C₁₀-Alkoxy, C₂-C₁₀-Alkenyl, C₂-C₁₀-Alkenyloxy, C₂-C₁₀-Alkinyl, C₂-C₁₀-Alkinyloxy
ein gesättigter oder ein- oder zweifach ungesättigter Ring, welcher neben Kohlenstoffatomen ein bis drei der folgenden Heteroatome als Ringglieder enthalten kann, der partiell oder vollständig halogeniert sein kann und/oder einen bis drei der folgenden Reste tragen kann: Nitro, Cyano, C₁-C₄-Alkyl, C₁-C₄-Alkoxy und C₁-C₄-Alkoxycarbonyl Sauerstoff, Schwefel und Stickstoff, und welcher direkt oder über ein Sauerstoff oder Schwefelatom an das Gerüst gebunden sein kann;
oder ein partiell oder vollständig halogenierter und/ oder durch einen bis drei der folgenden Reste: Nitro, Cyano, C₁-C₄-Alkyl, C₁-C₄-Alkoxy und C₁-C₄-Alkoxycarbonyl substituierter ein- oder zweikerniger aromatischer Rest, welcher neben Kohlenstoffatomen ein bis vier Stickstoffatome oder ein oder zwei Stickstoffatome und ein oder zwei Sauerstoff- oder Schwefelatome oder ein Sauerstoff- oder ein Schwefelatom als Ringglieder enthalten kann.

2. Verfahren zur Herstellung der Verbindungen der Formel I gemäß Anspruch 1, **dadurch gekennzeichnet, daß** man ein Benzylderivat der Formel II 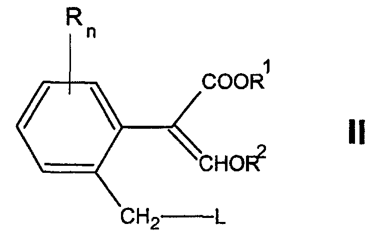 in der L eine nucleophil austauschbare Gruppe bedeutet, in Gegenwart einer Base mit einem Hydroxyazol der Formel III
HO―R₃ **III**
umsetzt.

3. Zur Bekämpfung von Schadpilzen geeignete Mischung, enthaltend einen inerten Zusatzstoff und eine wirksame Menge einer Verbindung der Formel I gemäß Anspruch 1.

4. Zur Bekämpfung von tierischen Schädlingen geeignete Mischung, enthaltend einen inerten Zusatzstoff und eine wirksame Menge einer Verbindung der Formel I gemäß Anspruch 1.

5. Verfahren zur Bekämpfung von Schadpilzen, **dadurch gekennzeichnet, daß** man die Schadpilze, ihren Lebensraum oder die von Schadpilzen freizuhaltenden Pflanzen, Flächen, Materialien oder Räume mit einer wirksamen Menge einer Verbindung der Formel I gemäß Anspruch 1 behandelt.

6. Verfahren zur Bekämpfung von tierischen Schädlingen, **dadurch gekennzeichnet, daß** man die Schädlingen, ihren Lebensraum oder die von Schädlingen freizuhaltenden Pflanzen, Flächen, Materialien oder Räume mit einer wirksamen Menge einer Verbindung der Formel I gemäß Anspruch 1 behandelt.

7. Verwendung der Verbindungen I gemäß Anspruch 1 zur Herstellung einer zur Bekämpfung von Schadpilzen geeigneten Mischung.

8. Verwendung der Verbindungen I gemäß Anspruch 1 zur Herstellung einer zur Bekämpfung von tierischen Schädlingen geeigneten Mischung.

9. Verwendung der Verbindungen I gemäß Anspruch 1 zur Bekämpfung von Schadpilzen.

10. Verwendung der Verbindungen I gemäß Anspruch 1 zur Bekämpfung von tierischen Schädlingen.

11. Verbindungen der Formel IV 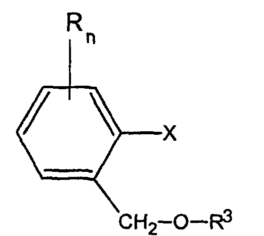 in der die Reste R und R³ sowie der Index n die in Anspruch 1 gegebene Bedeutung haben und X für CH₂CN, CH₂CO₂R und C(=O)CO₂R steht, wobei R die in Anspruch 1 gegebene Bedeutung hat.

## Claims

1. A compound of the formula I 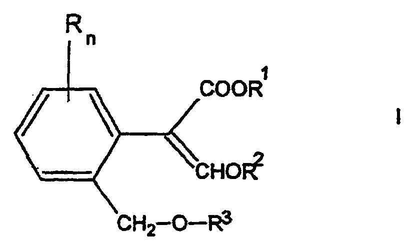 in which the index and the substituents are as defined below:
n is 0, 1, 2, 3 or 4, where the substituents R may be different and n is greater than 1;
R is nitro, cyano, halogen,
C₁-C₁₀-alkyl, C₂-C₁₀-alkenyl, C₂-C₁₀-alkynyl, C₁-C₁₀-alkoxy, C₂-C₁₀-alkenyloxy, C₂-C₁₀-alkynyloxy
where the aliphatic groups may be partially or fully halogenated and/or may carry one to three, in particular one, of the following radicals:
nitro, cyano, C₁-C₄-alkoxy, C₁-C₄-alkoxycarbonyl or a group =N-OR^{x} in which R^{x} is C₁-C₆-alkyl, C₃-C₆-alkenyl or C₃-C₆-alkynyl,
or a mono- or bicyclic aromatic ring system which, in addition to carbon atoms, may contain one to four nitrogen atoms or one or two nitrogen atoms and one oxygen or sulfur atom or one oxygen or one sulfur atom as ring members,
which for its part may be partially or fully halogenated and/or may carry one to three of the following radicals: nitro, cyano, C₁-C₄-alkyl, C₁-C₄-alkoxy and C₁-C₄-alkoxycarbonyl;
R¹, R² are C₁-C-alkyl [sic];
R³ is a triazole radical of the formula A 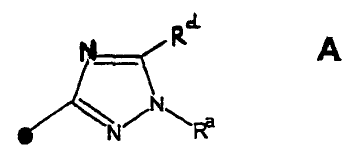
where the bond marked • corresponds to the bond to oxygen and where the substituents are as defined below:
R^{a} is C₁-C₁₀-alkyl, C₂-C₁₀-alkenyl or C₂-C₁₀-alkynyl;
where the aliphatic groups and/or [lacuna] to three, in particular one, of the following radicals:
nitro, cyano, C₁-C₄-alkoxy, C₁-C₄-alkoxycarbonyl or a group =N-OR^{x} in which R^{x} is C₁-C₆-alkyl, C₃-C₆-alkenyl or C₃-C₆-alkynyl,
or a mono- or bicyclic aromatic ring system which, in addition to carbon atoms, may contain one to four nitrogen atoms or one or two nitrogen atoms and one or two oxygen or sulfur atoms or one oxygen or one sulfur atom as ring members,
which for its part may be partially or fully halogenated and/or may carry one to three of the following radicals: nitro, cyano, C₁-C₄-alkyl, C₁-C₄-alkoxy and C₁-C₄-alkoxycarbonyl;
R^{d} is hydrogen, cyano, halogen,
C₁-C₁₀-alkyl, C₁-C₁₀-alkoxy, C₂-C₁₀-alkenyl, C₂-C₁₀-alkenyloxy, C₂-C₁₀-alkynyl, C₂-C₁₀-alkynyloxy,
a saturated or mono- or diunsaturated ring which, in addition to carbon atoms, may contain one to three of the following heteroatoms as ring members [lacuna], which may be partially or fully halogenated and/or may carry one to three of the following radicals:
nitro, cyano, C₁-C₄-alkyl, C₁-C₄-alkoxy and C₁-C₄-alkoxycarbonyl oxygen, sulfur and nitrogen [sic], and which may be attached to the skeleton directly or via an oxygen or sulfur atom;
or a mono- or bicyclic aromatic radical which, in addition to carbon atoms, may contain one to four nitrogen atoms or one or two nitrogen atoms and one or two oxygen or sulfur atoms or one oxygen or one sulfur atom as ring members and which is partially or fully halogenated and/or substituted by one to three of the following radicals: nitro, cyano, C₁-C₄-alkyl, C₁-C₄-alkoxy and C₁-C₄-alkoxycarbonyl.

2. A process for preparing the compounds of the formula I as claimed in claim 1, which comprises reacting a benzyl derivative of the formula II 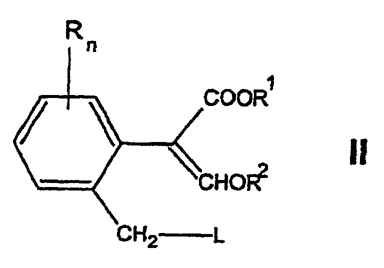 in which L is a nucleophilically exchangeable group, in the presence of a base with a hydroxyazole of the formula III
HO―R₃ **III**

3. A mixture suitable for controlling harmful fungi, which comprises an inert additive and an effective amount of a compound of the formula I as claimed in claim 1.

4. A mixture suitable for controlling animal pests, which comprises an inert additive and an effective amount of a compound of the formula I as claimed in claim 1.

5. A method for controlling harmful fungi, which comprises treating the harmful fungi, their habitat or the plants, areas, materials or spaces to be kept free from harmful fungi with an effective amount of a compound of the formula I as claimed in claim 1.

6. A method for controlling animal pests, which comprises treating the pests, their habitat or the plants, areas, materials or spaces to be kept free from pests with an effective amount of a compound of the formula I as claimed in claim 1.

7. The use of the compounds I as claimed in claim 1 for preparing a mixture suitable for controlling harmful fungi.

8. The use of the compounds I as claimed in claim 1 for preparing a mixture suitable for controlling animal pests.

9. The use of the compounds I as claimed in claim 1 for controlling harmful fungi.

10. The use of the compounds I as claimed in claim 1 for controlling animal pests.

11. A compound of the formula IV 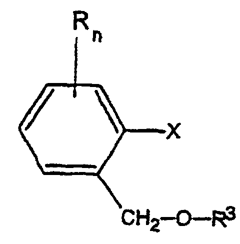 in which the radicals R and R³ and the index n are as defined in claim 1 and X is CH₂CN, CH₂CO₂R or C(=O)CO₂R, where R is as defined in claim 1 [sic].

## Revendications

1. Composés de formule générale I 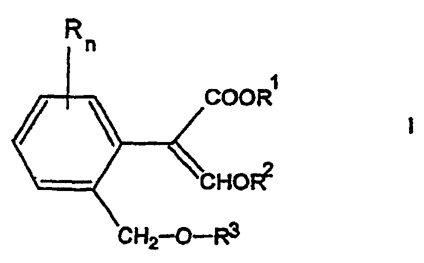 dans laquelle l'exposant et les substituants ont la signification suivante :
n 0, 1, 2, 3 ou 4, les substituants R pouvant être différents lorsque n est supérieur à 1 ;
R nitro, cyano, halogène,
alkyle en C1-C10, alkényle en C2-C10, alkinyle en C2-C10, alcoxy en C1-C10, alkényloxy en C2-C10, alkinyloxy en C2-C10,
les groupes aliphatiques pouvant être partiellement ou entièrement halogénés et/ou porter un à trois, notamment un des radicaux suivants :
nitro, cyano, alcoxy en C1-C4, alcoxycarbonyle en C1-C4 ou un groupe =N-ORx dans lequel Rx représente un alkyle en C1-C6, un alkényle en C3-C6 et un alkinyle en C3-C6,
ou une combinaison cyclique aromatique mono- ou bicyclique,
laquelle peut renfermer comme termes cycliques un à quatre atomes d'azote ou un ou deux atomes d'azote et un atome d'oxygène ou de soufre ou un atome d'oxygène ou un atome de soufre en plus des atomes de carbone,
laquelle peut également être partiellement ou entièrement halogénée et/ou porter un à trois des radicaux suivants : nitro, cyano, alkyle en C1-C4, alcoxy en C1-C4 et alcoxycarbonyle en C1-C4 ;
R1,R2 alkyle en C1-C4 ;
R3 un radical triazole de formule A 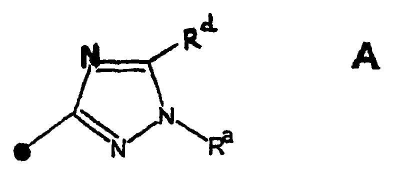
dans laquelle la liaison **caractérisée par** • correspond à la liaison avec l'oxygène et dans lesquelles les substituants ont la signification suivante :
Ra alkyle en C1-C10, alkényle en C2-C10 ou alkinyle en C2-C10 ;
les groupes aliphatiques pouvant porter un à trois, notamment un des radicaux suivants :
nitro, cyano, alcoxy en C1-C4, alcoxycarbonyle en C1-C4 ou un groupe =N-ORx dans lequel Rx représente un alkyle en C1-C6, un alkényle en C3-C6 et un alkinyle en C3-C6,
ou une combinaison cyclique aromatique mono- ou bicyclique,
laquelle peut renfermer comme termes cycliques un à quatre atomes d'azote ou un ou deux atomes d'azote et un ou deux atomes d'oxygène ou de soufre ou un atome d'oxygène ou un atome de soufre en plus des atomes de carbone,
laquelle peut également être partiellement ou entièrement halogénée et/ou porter un à trois des radicaux suivants : nitro, cyano, alkyle en C1-C4, alcoxy en C1-C4 et alcoxycarbonyle en C1-C4;
Rd hydrogène, cyano, halogène,
alkyle en C1-C10, alcoxy en C1-C10, alkényle en C2-C10, alkényloxy en C2-C10, alkinyle en C2-C10, alkinyloxy en C2-C10,
un composé cyclique saturé ou mono- ou bi-insaturé, lequel peut comprendre comme termes cycliques un à trois des hétéroatomes suivants en plus des atomes de carbone, être partiellement ou entièrement halogéné et/ou porter un à trois des radicaux suivants :
nitro, cyano, alkyle en C1-C4, alcoxy en C1-C4 et alcoxycarbonyle en C1-C4, oxygène, soufre et azote, et lequel peut être lié à la structure directement ou par l'intermédiaire d'un atome d'oxygène ou de soufre ;
ou un radical aromatique mono- ou bicyclique partiellement ou entièrement halogéné et/ou substitué par un à trois des radicaux suivants : nitro, cyano, alkyle en C1-C4, alcoxy en C1-C4 et alcoxycarbonyle en C1-C4, lequel peut comprendre comme termes cycliques un à quatre atomes d'azote ou un ou deux atomes d'azote et un ou deux atomes d'oxygène ou de soufre ou un atome d'oxygène ou un atome de soufre en plus des atomes de carbone.

2. Procédé pour la préparation des composés de formule I selon la revendication 1, **caractérisé en ce qu'**un dérivé du benzyle de formule II 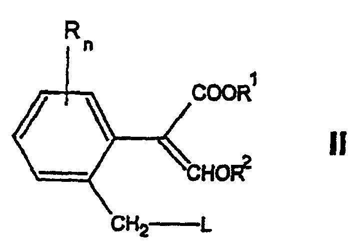 dans laquelle L représente un groupement substituable par nucléophilie, est transformé en présence d'une base avec un hydroxyazole de la formule III.
**HO―R**_{**3**} **III**

3. Mélange propre à la lutte contre des champignons nuisibles, contenant un adjuvant inerte et une quantité efficace d'un composé de la formule I selon la revendication 1.

4. Mélange propre à la lutte contre des parasites animaux, contenant un adjuvant inerte et une quantité efficace d'un composé de la formule I selon la revendication 1.

5. Procédé de lutte contre des champignons nuisibles **caractérisé en ce qu'**on traite les champignons nuisibles, leur habitat ou les plantes, surfaces, matières ou espaces à protéger des champignons avec une quantité efficace d'un composé de la formule I selon la revendication 1.

6. Procédé de lutte contre des parasites animaux **caractérisé en ce qu'**on traite les parasites animaux, leur habitat ou les plantes, surfaces, matières ou espaces à protéger des parasites avec une quantité efficace d'un composé de la formule I selon la revendication 1.

7. Utilisation des composés I selon la revendication 1 pour la préparation d'un mélange propre à la lutte contre des champignons nuisibles.

8. Utilisation des composés I selon la revendication 1 pour la préparation d'un mélange propre à la lutte contre des parasites animaux.

9. Utilisation des composés I selon la revendication 1 pour la lutte contre des champignons nuisibles.

10. Utilisation des composés I selon la revendication 1 pour la lutte contre des parasites animaux.

11. Composés de formule IV 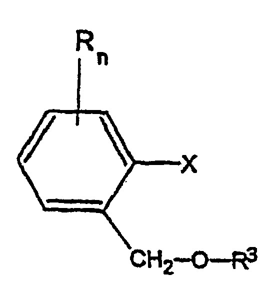 dans laquelle
les radicaux R et R3 ainsi que l'exposant n ont la signification indiquée dans la revendication 1 et X représente CH2CN, CH2CO2R et C(=O)CO2R, R ayant la signification indiquée dans la revendication 1.
